## Europäisches Patentamt
## European Patent Office
## Office européen des brevets

(19)

(11) Veröffentlichungsnummer : **0 134 025**
**B1**

(12) **EUROPÄISCHE PATENTSCHRIFT**

(45) Veröffentlichungstag der Patentschrift :
**15.06.88**

(51) Int. Cl.⁴ : **C 08 L101/04, C 08 L 1/00,**
**C 08 L 5/02, C 08 L 29/04,**
**C 08 L 77/00, C 08 L 89/00,**
**C 12 N 11/02, C 12 N 11/12,**
**G 01 N 33/53, D 01 D 5/00**

(21) Anmeldenummer : **84109485.7**

(22) Anmeldetag : **09.08.84**

(54) **Makromolekulare Massen mit chemisch aktiven Füllstoffen, Verfahren zu ihrer Herstellung und ihre Verwendung.**

(30) Priorität : 09.08.83 DD 253821
24.04.84 DD 262265
24.04.84 DD 262264
24.04.84 DD 262263
24.04.84 DD 262260
24.04.84 DD 262261

(43) Veröffentlichungstag der Anmeldung :
**13.03.85 Patentblatt 85/11**

(45) Bekanntmachung des Hinweises auf die Patenterteilung : **15.06.88 Patentblatt 88/24**

(84) Benannte Vertragsstaaten :
**AT BE CH DE FR GB IT LI NL SE**

(56) Entgegenhaltungen :
WO-A-79 /006 09
DE-A- 1 768 798
GB-A- 1 183 259

(73) Patentinhaber : **Akademie der Wissenschaften der DDR**
**Otto-Nuschke-Strasse 22/23**
**DDR-1086 Berlin (DD)**

(72) Erfinder : **Hunger, Hans-Dieter, Dr.**
**Zelterstrasse 84**
**DDR-1297 Zepernick (DD)**
Erfinder : **Coutelle, Charles, Prof. Dr.**
**Guddorfstrasse 22**
**DDR-1130 Berlin (DD)**
Erfinder : **Lampe, Jochen, Dr. sc. med., Arzt.**
**Robert-Rössle-Strasse 1**
**DDR-1115 Berlin (DD)**
Erfinder : **Schlechte, Horst, Dr. rer. nat.**
**Brennerstrasse 34**
**DDR-1100 Berlin (DD)**
Erfinder : **Schössler, Werner, Dr. rer. nat.**
**Rathenaustrasse 12**
**DDR-1281 Neu-Buch (DD)**
Erfinder : **Leiser, Robert-Matthias, Dr. rer. nat.**
**Otto-Sander-Strasse 2**
**DDR-4320 Aschersleben (DD)**
Erfinder : **Schubert, Jörg, Dr. rer. nat.**
**Pfeilergraben 69**
**DDR-4320 Aschersleben (DD)**
Erfinder : **Barchend, Gudrun, Dr. rer. nat.**
**Staakener Weg 8**
**DDR-4500 Dessau (DD)**

(74) Vertreter : **Patentanwälte Beetz sen. - Beetz jun.**
**Timpe - Siegfried - Schmitt-Fumian**
**Steinsdorfstrasse 10**
**D-8000 München 22 (DE)**

EP 0 134 025 B1

## Beschreibung

Die Erfindung bezieht sich auf makromolekulare Massen mit chemisch aktiven Füllstoffen, Verfahren zu ihrer Herstellung und ihre Verwendung.

An Makromoleküle mit nucleophilen Gruppen lassen sich Reagentien binden, die die chemische Aktivität dieser Moleküle bedeutend steigern.

Es sind bereits chemisch aktive makromolekulare Verbindungen bekannt, die z. B. OH-Gruppen oder $NH_2$-Gruppen aufweisen, an die 2,4,6-Trichlor-1,3,5-triazin (Cyanurchlorid) gebunden werden kann. Bei entsprechender Reaktionsführung bleiben zwei reaktive Chloratome des Cyanurchlorids erhalten. Diese beiden Atome sind für die besondere chemische Aktivität des Makromoleküls verantwortlich, da an dieser Stelle eine nucleophile Substitution durch entsprechende Verbindungen möglich ist. In DE-A-1 768 798 ist ein Verfahren zur Herstellung solcher Makromoleküle beschrieben.

Diese Makromoleküle bzw. die daraus hergestellten Produkte zeigen jedoch eine Aktivität der Chloratome, die der theoretischen Aktivität nur nahekommt. Dies ist im wesentlichen darauf zurückzuführen, daß die verbleibenden Chloratome am Triazinring leicht hydrolysierbar sind bzw. Vernetzungsreaktionen zwischen den einzelnen Makromolekülen stattfinden. Ein weiterer Nachteil der kovalenten Bindung von Cyanurchlorid an eine nucleophile Gruppe eines Makromoleküls zwecks Herstellung eines chemisch aktiven Polymers ist die nur begrenzt zur Verfügung stehende Anzahl der umsetzbaren Gruppen.

Der Erfindung liegt die Aufgabe zugrunde, die chemische Aktivität von überwiegend aus makromolekularen Stoffen bestehenden Mischungen bzw. Formkörpern auf Triazinderivat-Basis erheblich zu steigern und makromolekulare Massen auf Triazinderivat-Basis zu entwickeln, die durch geeignete Füllstoffe eine stark gesteigerte Bindungskapazität für Biomakromoleküle, Mikroorganismen und niedermolekulare Verbindungen aufweisen, sowie ein entsprechendes Herstellungsverfahren und ihre Verwendung anzugeben.

Die Aufgabe wird anspruchsgemäß gelöst. Vorteilhafte Weiterbildungen sind Gegenstand der abhängigen Ansprüche.

Die erfindungsgemäßen makromolekularen Massen mit chemisch aktiven Füllstoffen bestehen aus

(I) 1 bis 80 Vol.-% einer makromolekularen Verbindung mit 4,6-Dihalogen-1,3,5-triazin-Gruppierungen,

(II) 0,5 bis 50 Vol.-% eines oder mehrerer 2,4,6-Trihalogen-1,3,5-triazine (Cyanurhalogenide),

(III) bis zu 20 Vol.-% eines oder mehrerer Halogenide und

(IV) 5 bis 80 Vol.-% einer makromolekularen Verbindung, die mit der makromolekularen Verbindung (I) sowie den Füllstoffen verträglich ist, sowie gegebenenfalls

(V) bis zu 5 Vol.-% Puffersubstanzen und gegebenenfalls

(VI) einem flüssigen Dispergiermittel.

Die Eigenschaften der Massen, wie etwa die mechanischen und physikalischen Eigenschaften, werden durch Menge und Art der eingesetzten Polymeren und durch ihre Formgebung bestimmt. Die chemische Aktivität ist in erster Linie durch die Menge der vorhandenen Füllstoffe, wie 2,4,6-Trihalogen-1,3,5-triazine II und Halogenide III, sowie ferner auch durch die Formgebung der Massen festgelegt. Das Cyanurhalogenid verfügt über drei aktive Halogenatome, die nucleophil substituiert werden können. Allerdings ist die außerordentlich hohe Aktivität der Massen nicht allein durch die erhöhte Anzahl von aktiven Halogenatomen erklärbar. Es zeigt sich überraschend, daß nur die Massen in ihrer erfindungsgemäßen Zusammensetzung diese hohen Aktivitätswerte aufweisen. Die in den Massen ebenfalls vorhandenen Halogenide, insbesondere Alkalihalogenide, dienen der Mikrofixierung des Cyanurhalogenids.

Als makromolekulare Verbindungen IV sind prinzipiell alle Polymeren (mit oder ohne nucleophile Gruppen) verwendbar, die mit der makromolekularen Verbindung I und den Füllstoffen verträglich sind. Das gleiche gilt für die makromolekulare Verbindung I, die ggfs. noch weitere nucleophile Gruppen aufweisen kann. Beispiele für geeignete Polymere sind Cellulose, Cellulosederivate, Dextrane und Gelatine, aber auch synthetische Polymere mit OH- und $NH_2$-Gruppen, wie Polyamide und Polyvinylalkohole.

Die bevorzugt eingesetzten Füllstoffe sind Cyanurchlorid als Triazinderivat II, Natriumchlorid als Halogenid III und Natriumacetat als Puffersubstanz V, das gleichzeitig als Füllstoff dient. Als Dispergiermittel VI eignen sich insbesondere wasserfreie inerte Lösungsmittel.

Je nach Wahl des Ausgangsmaterials und je nach Verfahrensdurchführung haben die erfindungsgemäßen Massen eine kompakte oder eine disperse Form. Bei Vorliegen einer dispersen Form können die Teilchen faserförmig, kugelförmig und/oder eckig sein. Bei Vorliegen einer kompakten Form können die Formkörper kugelförmig, flächig oder eckig bzw. kantig sein.

Die Formgebung der Masse beeinflußt insofern die chemische Aktivität, als eine große Oberfläche mehr Stellen für die Fixierung von Biomakromolekülen bietet. Demzufolge ist ein disperses System besonders geeignet, nucleophile Reagentien zu binden. Faserförmige Teilchen lassen sich beispielsweise zu Papier formen, wenn als Polymere Cellulose und Celluloseverbindungen eingesetzt werden. In diesem Fall kann der Zusatz eines anderen Polymers, wie z. B. eines Polyamids, dem Papier zusätzliche mechanische Stabilität verleihen. Die Massen können in festem oder flüssigem Aggregatzustand vorliegen, wobei der Aggregatzustand zum einen durch den Aggregatzustand des Polymeranteils und zum anderen durch ein flüssiges Dispergiermittel, das ggfs, teilweise auch die Funktion eines Lösungsmittels hat, bestimmt wird.

Bei der Herstellung der erfindungsgemäßen Massen und entsprechender Formkörper ist zu beachten, daß in der Regel die eingesetzten Füllstoffe (II, III, V) in einer Lösung vorliegen müssen, was eine genaue Abstimmung der Mengen der Komponenten erfordert. Die Polymerkomponenten liegen zweckmäßigerweise in dispersem Zustand vor, wobei die Teilchenform und Teilchengröße im wesentlichen vom Verwendungszweck abhängen. Allerdings ist es möglich, daß auch die Polymerkomponenten teilweise in gelöster Form vorliegen. Durch die Einhaltung der im folgenden beschriebenen grundsätzlichen Verfahrensbedingungen zur Herstellung der Massen bzw. der Formkörper kommt es zur gleichzeitigen Auskristallisation und Abscheidung der Füllstoffe auf der Polymerphase.

Die Herstellung der erfindungsgemäßen Massen kann nach verschiedenen Verfahrensvarianten erfolgen :

1. Variante

Die makromolekulare Verbindung I mit 4,6-Dihalogen-1,3,5-triazin-Gruppierungen wird in einer Rührvorrichtung mit einem Dispergiermittel, dem Triazin II, dem Halogenid III und der makromolekularen Verbindung IV versetzt und gründlich durchmischt, worauf das Dispergiermittel abgedampft wird. Dabei kommt es zur mikrokristallinen Abscheidung der Füllstoffe II, III in der Mischung, insbesondere an der Polymerphase. Die erhaltene Masse wird mit einem Dispergiermittel gewaschen, dann getrocknet und feuchtigkeitsdicht verpackt.

2. Variante

Das Halogenid III und das Triazin II werden durch Zusatz geeigneter Lösungsmittelgemische, wie mit Wasser mischbaren Lösungsmitteln (Alkoholen, Ketonen, organischen Säuren), auf den makromolekularen Verbindungen I und IV mikrokristallin abgeschieden. Bevorzugt wird 2,4,6-Trichlor-1,3,5-triazin auf Cellulosepartikeln fixiert, indem die Cellulosepartikel zunächst mit 2,4,6-Trichlor-1,3,5-triazin (Cyanurchlorid) behandelt werden, das Cyanurchlorid unmittelbar darauf mit einer Mischung aus Essigsäure und einem mit Wasser mischbaren organischen Lösungsmittel bzw. Lösungsmittelgemisch mit 50 bis 90 Vol.-% organischem Lösungsmittel fixiert wird und die Cellulosepartikel anschließend mit einem mit Wasser mischbaren organischen Lösungsmittel bzw. Lösungsmittelgemisch gewaschen werden.

Als mit Wasser mischbare Lösungsmittel werden primäre Alkohole, wie Methanol, Ethanol, Propanol und Butanol, einschließlich ihrer Isomeren, und mit Wasser mischbare Ketone, wie Aceton, verwendet.

3. Variante

Die makromolekulare Verbindung IV wird nach Abdampfen des Dispergiermittels und Waschen in Form einer Dispersion oder Lösung zugesetzt ; während der Durchmischung wird die flüssige Phase abgedampft.

Die Herstellung von Formkörpern erfolgt prinzipiell auf zwei Wegen :

1. Die hergestellte Masse wird durch an sich bekannte Verfahren einem Formungsprozeß unterzogen, der sich im wesentlichen nach den eingesetzten Polymeren und dem Verwendungszweck richtet. Beispielsweise muß beim Verspinnen der Masse ein spinnbares Polymer enthalten sein. Die Verformbarkeit der Masse wird durch Lösungsmittel- bzw. Dispergiermittelzusatz erreicht, die ggfs. durch Trocknung entfernt werden.

2. Man geht von bereits vorgeformten porösen Polymerkörpern aus, die mit einer Lösung behandelt werden, die das Triazin II und das Halogenid III enthält.

Zweckmäßigerweise wird die Behandlung unter Erzeugung einer intensiven Turbulenz im Behandlungsbad durchgeführt. Dabei kommt es zu einer mikrokristallinen Abscheidung auf der polymeren Phase. Auf diese Weise können hochaktive Formkörper mit papierähnlicher Matrix hergestellt werden.

Die erfindungsgemäßen Massen und insbesondere Formkörper daraus sind beispielsweise in der Molekularbiologie z. B. für Kapillar- und Elektroblotting und für Screening-Tests zur Bindung von Mikroorganismen, in Festphasen-Immunoassays sowie etwa als Träger für Teststreifen vorteilhaft verwendbar.

Die erfindungsgemäßen Massen und die daraus hergestellten Formkörper zeichnen sich durch außergewöhnlich hohe Bindungskapazität gegenüber Biomakromolekülen, Mikroorganismen und niedermolekularen Verbindungen, wie Farbstoffen und Leukofarbstoffen, aus Formkörper aus makromolekularen Massen mit papierähnlicher Matrix beispielsweise haben eine Bindungskapazität für DNS bzw. RNS, die etwa 10-fach höher ist als die vergleichbarer Papiere. Die Bindungskapazität kann durch die Zusammensetzung variiert werden, wobei die Spitzenwerte bei etwa 10 000 µg DNS/g Gemisch liegen.

3

Diese hohe Aktivität ist nicht allein durch das Vorhandensein von kovalent gebundenem Triazin bzw. von freiem Triazin erklärbar ; sie wird in kooperativer Weise durch die erfindungsgemäße Zusammensetzung der Gemische bewirkt.

Die erfindungsgemäßen Massen sind insbesondere in der Molekularbiologie vorteilhaft verwendbar. So kann z. B. ein erfindungsgemäßes Flachmaterial auf Cellulosebasis, das ggfs. Polyamidfasern enthält, bei Transfertechniken (Southern-, Northern-, Westerntechnik) eingesetzt werden. Bei diesen Techniken wird z. B. das in einem Gel elektrophoretisch aufgetrennte Nucleinsäure- bzw. Proteingemisch auf das Flachmaterial als Träger übertragen und fixiert und steht dann für spezifische Nachweisreaktionen zur Verfügung. Der Transfer der Verbindungen aus den Trenngelen kann dabei z. B. durch Kapillar- oder Elektroblotting erfolgen. Mit großem Vorteil können die erfindungsgemäßen Massen bzw. Formkörper ferner für Papierteststreifen eingesetzt werden, um eine feste Bindung der verwendeten Nachweisreagentien im Papier zu erreichen, so daß keine Auswascheffekte auftreten.

Die Festphasen-Immunoassays unter Verwendung der erfindungsgemäßen makromolekularen Massen mit chemisch aktiven Füllstoffen werden zur Bestimmung von Antigenen, Antikörpern, Haptenen, Viren, Hormonen, Pharmaka etc. eingesetzt.

Unter Fixierung der komplementären Antikörper oder Antigene an cellulosehaltigen Materialien als feste Phase, Durchführung der Immunreaktion, Spaltung der gebildeten Antigen-Antikörper-Komplexe durch die Dissoziation herbeiführender Reagentien und Wiedereinsatz der beladenen festen Phase im Immunoassay werden als feste Phase Formkörper verwendet, die aus Cellulose und/oder Cellulosederivaten mit 4,6-Dichlor-1,3,5-triazin-Gruppierungen in einer Menge von 1 bis 80 Vol.-%, 2,4,6-Trihalogen-1,3,5-triazin in einer Menge von 0,5 bis 50 Vol.-%, Chloriden in einer Menge bis zu 20 Vol.-% und Cellulose und/oder Cellulosederivaten in einer Menge von 5 bis 80 Vol.-% bestehen.

Die Formkörper sind dabei zweidimensional oder dreidimensional und für die oberflächliche Beladung mit Antigenen und Antikörpern nutzbar.

Die als feste Phase verwendeten Formkörper können unterschiedliche geometrische Form besitzen. Besonders geeignet sind halbkugelförmige, kugelförmige, planare, würfelförmige oder zylindrische Körper. Das Volumen der Körper beträgt vorzugsweise mindestens 0,01 ml und sollte 15 ml nicht überschreiten. Bei flächigen Formkörpern ist weniger das Volumen als eine jeweils reproduzierbare, verfügbare Fläche von Bedeutung. Diese nicht größer als sollte 0,001 cm$^2$ nicht unterschreiten und 5 cm$^2$ sein. Die Formkörper sind kompakt oder hohl, wobei die Hohlräume geschlossen oder offen sind. Die Cellulose bzw. die Cellulosederivate werden teilweise oder vollständig in Form faserförmiger Partikel eingesetzt. Ein besonders zweckmäßig ausgeführter Formkörper ist ein Flächenträger mit papierähnlicher Matrix.

Bei Festphasen-Immunoassays erreicht man aufgrund der hohen Beladungskapazität von aus erfindungsgemäßen Massen hergestellten Formkörpern, wie Flachmaterialien, als Träger ausgezeichnete Ergebnisse :
— Durch die Bindung von Antigenen bzw. Antikörpern an die erfindungsgemäßen Formkörper lassen sich wesentlich höhere Beladungsdichten als bisher erzielen ;
— durch die Immobilisierung treten keine Desorptionen des Immunreaktanden während der notwendigen Inkubations- und Waschvorgänge beim Immunoassay auf. Dadurch wird der methodische Fehler erheblich verringert.

Die Verwendung der erfindungsgemäßen Materialien führt zu einer erheblichen Reduzierung der Kosten in der klinischen Praxis. Einmal werden Einweggefäße aus Kunststoffmaterialien entbehrlich, zum anderen werden Einsparungen an mitunter sehr wertvollen und teuren Proteinen ersieht. Durch die Immobilisierung von Antigenen bzw. Antikörpern auf erfindungsgemäßen Formkörpern ergeben sich neue Aspekte der Fertigung einfach zu handhabender und standardisierter Testkits für kommerzielle Hersteller.

Die erfindungsgemäßen Trägermaterialien eignen sich ferner günstig zur Fixierung von Mikroorganismen, wobei überraschenderweise ihre Lebens- und Vermehrungsfähigkeit erhalten bleiben. Eine weitere wichtige Verwendbarkeit des Trägermaterials unter Ausnutzung seiner hohen Bindungskapazität besteht in der Möglichkeit des Screenings von Substanzen aus ungereinigten biologischen Proben ; hierzu gehören z. B. der Nachweis von viralen Ribonucleinsäuren, freien viralen Nucleinsäuren (RNS) und replikativen Formen der Virus-RNS, von Viroiden und Virusoiden in aus Pflanzen oder anderem Material bzw. Säften gewonnenen Extrakten durch Bindung der nachzuweisenden RNS an den erfindungsgemäßen Flachmaterialien als Trägern und nachfolgende Hybridisierung mittels markierter komplementärer Desoxyribonucleinsäure (cDNS), klonierter cDNS (Rekombinant cDNS), doppelsträngiger RNS (dsRNS), einsträngiger Virionen-RNS (ssRNS) bzw. komplementärer RNS (cRNS) und der RNS replikativer Intermediates als molekulare Hybridisierungssonden.

Der Träger besteht aus Cellulose und/oder Cellulosederivaten mit 4,6-Dichlor-1,3,5-triazin-Gruppierungen in einer Menge von 1 bis 80 Vol.-%, 2,4,6-Trichlor-1,3,5-triazin in einer Menge von 0,5 bis 50 Vol.-%, Halogeniden in einer Menge bis zu 20 Vol.-%, Cellulose und/oder Cellulosederivaten in einer Menge von 5 bis 80 Vol.-% und ggfs. Puffersubstanzen in einer Menge bis zu 5 Vol.-%

Der Flächenträger besitzt eine wesentlich höhere RNS-Bindungskapazität als die anderen beschriebenen Trägersysteme und ermöglicht dadurch die Analyse von ungereinigten Pflanzenextrakten.

Die Verwendung zur Herstellung von Papierteststreifen erfolgt für den Nachweis von organischen und anorganischen Bestandteilen in Flüssigkeiten.

Es wird ein flächiger Träger verwendet, der aus Cellulose, und/oder Cellulosederivaten mit 4,6-Dichlor-1,3,5-triazin-Gruppierungen in einer Menge von 1 bis 80 Vol.-%, 2,4,6-Trichlor-1,3,5-triazin in einer Menge von 0,5 bis 50 Vol.-%, Chloriden in einer Menge bis zu 20 Vol.-% und Cellulose und/oder Cellulosederivaten in einer Menge von 5 bis 80 Vol.-% besteht. Die eingesetzte Cellulose bzw. das Cellulosederivat bestehen teilweise oder vollständig aus faserförmigen Partikeln. Die Herstellung der Teststreifen erfolgt dadurch, daß flächige Träger, insbesondere mit papierähnlicher Matrix, mit einer Lösung, die die Reagentien enthält, imprägniert werden.

Die Erfindung wird im folgenden anhand von Ausführungsbeispielen näher erläutert.

## Beispiel 1

Ein Gemisch von 10 Volumenteilen Cellulosefasern und 70 Volumenteilen Cellulosefasern mit Dichlortriazingruppierungen wird in einem Rührwerk mit einer Mischung aus einer gesättigten Kochsalzlösung (9 Volumenteile NaCl) in Wasser und einer Lösung von 11 Volumenteilen Cyanurchlorid in Aceton verrührt. Nach Zugabe von Essigsäure werden die Lösungsmittel im Vakuum abgedampft, so daß es zu einer mikrokristallinen Abscheidung des Gemischs auf den Cellulosefasern kommt. Durch Rühren während der Kristallisation wird die Kristallisation beschleunigt, und es kommt zur Ausbildung relativ kleiner Kristalle.

Die Kristalle können im Rasterelektronenmikroskop sichtbar gemacht werden und besitzen eine Größe bis zu 100 μm, wobei die Größe der Kristalle durch die Kristallisationsbedingungen variierbar ist. Die optimale Größe liegt zwischen 1 und 10 μm.

Nach Beendigung der Kristallisation werden die Cellulosefasern 2 Mol/10 min mit Aceton gewaschen und dann im Vakuum getrocknet.

Zur sicheren Aufbewahrung und zur weiteren Verarbeitung zu Formkörpern kann das erhaltene Gemisch in wasserfreiem Dioxan dispergiert werden. Das Gemisch zeigt eine DNS-Bindungskapazität von ca. 10 000 μg DNS/g.

Der DNS-Bindungstest wird folgendermaßen durchgeführt: Eine bestimmte Menge des aktiven Gemischs bzw. eine bestimmte Fläche entsprechender Formkörper werden in einem Phosphatpuffer pH 5,5 mit einem Überschuß an radioaktiv markierter denaturierter, beschallter DNS über Nacht bei Raumtemperatur inkubiert. Nach Waschen mit Wasser wird die gebundene DNS-Menge durch Messung der Radioaktivität des Trägers bestimmt.

Die DNS-Bindungskapazität wird auf 1 g des Gemischs bzw. bei Formkörpern auf 1 cm² Fläche bezogen.

## Beispiel 2

Ein Gemisch aus Polyamidfasern und Polyamidfasern mit Dichlortriazingruppierungen wird in einem Rührwerk mit einer Mischung aus einer gesättigten Kochsalzlösung in Wasser und einer Lösung von Cyanurbromid in Aceton verrührt. Die weitere Verarbeitung erfolgt wie in Beispiel 1.

## Beispiel 3

Ein Gemisch von Polyurethan (Pulver oder Fasern) und Cellulosefasern mit Dichlortriazingruppierungen wird in einem Rührwerk mit einer Mischung aus einer gesättigten Kochsalzlösung und einer Cyanurchloridlösung in Aceton verrührt. Die weitere Verarbeitung erfolgt wie in Beispiel 1.

## Beispiel 4

Ein Gemisch von DEAE-Cellulosefasern und Cellulosefasern mit Dichlortriazingruppierungen wird in einem Rührwerk mit einer Mischung aus einer gesättigten Kochsalzlösung in Wasser und einer Lösung von Cyanurchlorid in Aceton verrührt. Die weitere Verarbeitung erfolgt wie in Beispiel 1.

## Beispiel 5

Ein Gemisch von Gelatine und Celluloseteilchen mit Dichlortriazingruppierungen wird in einem Rührwerk mit einer Mischung aus einer gesättigten Kochsalzlösung in Wasser und einer Lösung von Cyanurchlorid in Aceton verrührt.
Die weitere Verarbeitung erfolgt wie in Beispiel 1.

## Beispiel 6

Ein Gemisch von Polyvinylalkohol und Cellulosefasern mit Dichlortriazingruppierungen wird in einem Rührwerk mit einer Mischung aus einer gesättigten Kochsalzlösung in Wasser und einer Lösung von Cyanurchlorid in Aceton verrührt.

Die weitere Verarbeitung erfolgt wie in Beispiel 1.

## Beispiel 7

Ein Gemisch von Cellulosefasern und Cellulosefasern mit Dichlortriazingruppierungen wird in einem Rührwerk mit einer Mischung aus einer gesättigten Kochsalzlösung in Wasser und einer Lösung von Cyanurchlorid in Aceton verrührt. Nach Zugabe von Essigsäure und 0,5 Vol.-% Natriumacetat (berechnet auf den gesamten Mischungsansatz) werden die Lösungsmittel abgedampft.
Die weitere Verarbeitung erfolgt wie in Beispiel 1.

## Beispiel 8

Cellulosefasern mit Dichlortriazingruppierungen werden wie in Beispiel 1 beschrieben mit Kochsalz- und Cyanurchloridlösung behandelt. Das erhaltene Gemisch wird anschließend in einer Lösung von Polyvinylchlorid (mittlere Molekülmasse 30 000) in Dioxan dispergiert und zu einem flächigen papierähnlichen Formkörper verarbeitet.

## Beispiel 9

Filterblättchen aus Cellulose werden nach bekannten Verfahren mit Cyanurchlorid aktiviert, z. B. durch 15 min Behandlung mit 3 N NaOH, Zwischentrocknen der Filter und anschließende Behandlung in einer Lösung von 5 % Cyanurchlorid in Dioxan/Xylol (1 : 1) während 10 min.
Nach Abschluß der Aktivierung wird durch Zusatz einer Mischung von 70-80 % Aceton und Eisessig eine mikrokristalline Abscheidung des NaCl und der Triazinkomponente erreicht. Die Filterblättchen werden anschließend 2 mol 10 min mit Aceton gewaschen und danach getrocknet.
Die DNS-Bindungskapazität der Filterblättchen erreicht 200 $\mu$g/cm$^2$.

## Beispiel 10

Filterblättchen aus Cellulose werden wie in Beispiel 9 mit Cyanurchlorid aktiviert. Nach Abschluß der Aktivierung wird durch Zusatz einer Mischung von Butanol/Aceton/Eisessig (50 : 30 : 20) eine mikrokristalline Abscheidung von NaCl und der Triazinkomponente erreicht.
Die Filterblättchen werden anschließend 2 Mol 10 min mit Aceton gewaschen und getrocknet.

## Beispiel 11

Filterblättchen aus Cellulose werden wie in Beispiel 9 mit Cyanurchlorid aktiviert.
Nach der Aktivierung werden die Filter in einer essigsauren Lösung von Cyanurchlorid und NaCl in einem Aceton/H$_2$O-Gemisch geschüttelt, worauf die Lösungsmittel im Vakuum abgedampft werden. Die Filter werden anschließend ggfs. 2 Mol 10 min mit Aceton gewaschen und getrocknet.

## Beispiel 12

Ein Flachmaterial mit papierähnlicher Matrix gemäß der Erfindung als Träger wird in 4 × 4 cm große Quadrate geschnitten.
Je Flächeneinheit werden 5 $\mu$l Puffer (0,1 M Phosphat, 0,9 % NaCl) mit verschiedenen Verdünnungen von Bacillus subtilis (168 M) in geordneter Reihenfolge aufgetropft.
Verdünnung 1 = 100 000 Keime
Verdünnung 2 =  10 000 Keime
Verdünnung 3 =   1 000 Keime
Verdünnung 4 =     100 Keime
Verdünnung 5 =      10 Keime
Verdünnung 6 =       1 Keim.
Dabei werden Phosphatpuffer des pH-Bereichs 5-8 getestet. Als Kontrolle werden die analogen Auftragungen auf normalem Filterpapier vorgenommen.
Nach der Auftragung erfolgt eine Inkubation von 10-20 min bei Raumtemperatur.
Danach wird der Träger mit der Auftropfseite nach unten auf eine Fritte gelegt und mit je 10 ml Phosphatpuffer pH 7,6 gewaschen.
Anschließend wird der Träger mit der Auftropfseite nach oben in Petrischalen auf Agar gelegt und mit 10 ml Weichagar bei 42 °C überschichtet (9-cm-Petrischalen, Glucose-Bouillon-Agar pH 7,2 ± 0,2). Die Inkubation erfolgt bei 37 °C im Brutschrank. Nach 36 h zeigt sich ein Koloniewachstum an der Auftropfstelle.
Das Vergleichspapier zeigt nur bei pH 7,0 Koloniewachstum bis Verdünnung 3, während der verwendete erfindungsgemäße Träger bei pH 6,5 Koloniewachstum bis Verdünnung 4 zeigt. Bei pH 7,0 zeigt der erfindungsgemäße Träger ein Koloniewachstum bis Verdünnung 6. Bei pH 7,6 zeigt nur der erfindungsgemäße Träger Koloniewachstum bei Verdünnung 1.

Die Ergebnisse zeigen, daß der erfindungsgemäße Träger bei pH 7,0 eine komplette Bindung bei voller Lebensfähigkeit der gebundenen Mikroorganismen aufweist. Die Bindung ist zwischen pH 6,5 und 7,0 gegenüber herkömmlichem Vergleichspapier mindestens 1 000-fach höher.

## Beispiel 13

Ein Flachmaterial mit papierähnlicher Matrix gemäß der Erfindung als Träger wird mit Human-Immunglobulin G, das mit $^{125}J$ radioaktiv markiert ist, in verschiedenen Konzentrationen und in Puffersystemen versetzt und 16 h bei 37 °C inkubiert. Nach dreimaligem Waschen mit PBS-Puffer, pH 7,4, der 0,05 % Tween® 20 enthält, werden die eventuell noch überschüssigen reaktiven Gruppen mit 1 m Ethanolaminlösung blockiert. Nach erneutem Waschen mit dem obigen PBS-Puffer wird die gebundene Proteinmenge durch Messung der Radioaktivität ermittelt. Zur Ermittlung möglicher Desorptionen werden die Proben anschließend erneut siebenmal mit PBS-Puffer gewaschen. Die so beladenen Träger werden danach zur Ermittlung der Immunreaktivität mit einem Konjugat bestehend aus einem Kaninchen-anti-Human-IgG und dem Enzym Alkalische Phosphatase versetzt und über Nacht bei Raumtemperatur inkubiert. Nach erneutem Waschen wird die Enzymaktivität der Alkalischen Phosphatase durch Hydrolyse von 4-Nitrophenylphosphat in 0,5 ml Diethanolaminpuffer pH 9,8 und photometrische Messung des Produkts bei 405 nm nach Stoppen mit 1 n NaOH bestimmt. Nach Ausführung des Enzymimmunoassays werden die Träger zweimal mit je 0,5 ml PBS-Puffer gewaschen und nachfolgend in Mehrfachbestimmungen mit einem der folgenden Dissoziationsreagentien für 1 h bei 4 °C versetzt: 1) PBS-Puffer, 2 M an NaCl, mit 5 % Dioxan; 2) 1 M KSCN; 3) 3 M KSCN; 4) 4,5 M $MgCl_2$; 5) 1 M NaJ; 6) HCl-Glycin-Puffer pH 2,8. Durch alle diese Agentien werden die gebundenen Antigen-Antikörper-Komplexe gespalten, so daß die mit Human-IgG beladenen Träger erneut im Enzymimmunoassay eingesetzt werden können.

## Beispiel 14

Flächige erfindungsgemäße Formkörper (F = 56 mm²) werden, wie in Beispiel 13 beschrieben, mit humanem Faktor VIII beladen. Die Träger werden dann in Polystyrol-Mikrotitrationsplatten oder Polystyrolröhrchen übergeführt, wonach ein Enzymimmunoassay zur Bestimmung von F-VIII-Antigen in der literaturbekannten Weise durchgeführt wird (W. Schößler, M. Stepanauskas, Chr. Dittrich, H. Heine, Acta biol. med. germ. 41 (1982) 263: W. Schößler, M. Stepanauskas, Chr. Dittrich, Acta biol. med. germ. 41 (1982) 695). Hierzu werden die Formkörper mit einem Inkubationsgemisch, bestehend aus dem zu bestimmenden Plasma und einem im Überschuß vorhandenen Kaninchen-anti-Human-Faktor-VIII-Antikörper, versetzt und 6 h bei 37 °C inkubiert. Nach erneutem Waschen mit PBS-Puffer, pH 7,4, der 0,05 % Tween® 20 enthält, werden 200 µl eines Konjugats, bestehend aus einem Hammel-anti-Kaninchen-IgG und dem Enzym Alkalische Phosphatase, zugegeben, worauf nach mehrstündiger Inkubation und nachfolgendem Auswaschen die Enzymaktivität, wie in Beispiel 13 beschrieben, bestimmt wird. Die Abspaltung der gebundenen Antikörper erfolgt mit einem der in Beispiel 13 angeführten Dissoziationsreagentien, so daß die mit dem Faktor VIII beladenen Träger erneut im Immunoassay eingesetzt werden können.

## Beispiel 15

Ein erfindungsgemäßes Flachmaterial als Träger mit papierähnlicher Matrix wird, wie in Beispiel 14 beschrieben, mit humanem Faktor VIII beladen. Dieser Träger dient im folgenden als Modell für Screening-Untersuchungen auf (monoklonale) Antikörper. Nach Ausführung der Waschvorgänge, die sich bei diesem Träger besonders einfach gestalten, werden die zu untersuchenden und zu bestimmenden Substanzen mit einem geeigneten Auftragestempel punktförmig aufgetragen. In diesem Fall werden hierzu geeignete Verdünnungen eines Kaninchen-anti-Human-Faktor-VIII-Antiserums bzw. eines Kaninchenserums als Negativkontrolle als Antikörperquelle verwendet. Nach 4-6 h Inkubation bei 37 °C wird der Träger erneut gewaschen und mit einem Schaf-anti-Kaninchen-IgG, gekoppelt mit dem Enzym Peroxidase, 4 h bei 37 °C oder über Nacht bei Raumtemperatur inkubiert und erneut mehrmals gewaschen. Anschließend wird die Enzymaktivität mit einem geeigneten Nachweissystem bestimmt, z. B. mit 4 mM o-Phenylendiamin und 1,5 mM $H_2O_2$ unter visueller Auswertung der entstehenden Färbung. Eine positive Färbung weist eindeutig auf einen Antikörper hin.

## Beispiel 16

(a) Nachweis von TRV-Virus (tobacco rattle virus) mit Rekombinant-cDNS in Kartoffeln

0,5 g Pflanzenmaterial werden in einer Reibschale unter Zusatz von 0,5 ml 0,1 M Tris-HCl (pH 7,5) homogenisiert. Das Homogenisat wird 5 min in einer Tischzentrifuge in 1,5-ml-Zentrifugenbechern zentrifugiert. 250 µl des Überstands werden mit 250 µl eines Gemischs aus 1 Teil Phenol mit 0,1 % 8-Hydroxychinolin, äquilibriert mit 0,1 M Tris-HCl (pH 7,5), 1 Teil Chloroformgemisch (24 Teile Chloroform: 1 Teil iso-Amylalkohol) gemischt, angereichert mit 1 % SDS und nach kräftigem Schütteln 5 min auf 65 °C erwärmt. Nach Brechen der Phasen durch Zentrifugieren wird die wäßrige Phase abgenommen, zweimal mit Chloroformgemisch ausgeschüttelt, durch Zugabe von konzentriertem Natriumacetat auf eine Endkonzentration von 0,3 M gebracht und mit 3 Volumina 96-%igem Alkohols versetzt. Die RNA wird durch 10 min Abkühlen des Probenröhrchens auf —70 °C ausgefällt. Der Alkohol wird anschließend abgesaugt ; die Probe wird danach zweimal mit 70-%igem Alkohol nachgewaschen und nach Abtrocknen des Alkohols in 4 $\mu$l destilliertem Wasser aufgenommen. Die Probe wird anschließend auf eine Endkonzentration von 1 M Glyoxal, 50 % DMSO und 10 mM $Na_{2/3}PO_4$ (pH 7,0) in einem Gesamtvolumen von 16 $\mu$l gebracht, wonach die RNS durch 1 h Erhitzen auf 50 °C und anschließendes Abschrecken im Eisbad denaturiert wird. 1 $\mu$l der denaturierten Probe wird mittels einer Mikroliterpipette auf ein erfindungsgemäßes Flachmaterial als Träger aufgetragen. Nach dem Auftragen aller Proben einschließlich Proben gesunder Kontrollpflanzen wird das Material 1 h bei Raumtemperatur fixiert. Die Absättigung der freien Bindungskapazität des Papiers erfolgt durch Behandlung mit 10 % Ethanolamin pH 7,0. Anschließend erfolgt eine Vorhybridisierung des beladenen Trägers in einer Lösung aus 50 % Formamid, 5-fach-Denhardt's Lösung, 0,1 % SDS und 5-fach-SSPE unter Zusatz von 10 $\mu$g/ml Vorhybridisierungslösung denaturierter unspezifischer DNS bei einer Temperatur von 42 °C. Nach 18 h wird die Lösung gegen eine Lösung aus 50 % Formamid, 2-fach-Denhardt's Lösung, 0,1 % SDS, 5-fach-SSPE und 10 $\mu$g/ml Hybridisierungslösung denaturierter unspezifischer DNS ausgetauscht. Diese Lösung enthält ebenfalls 1 $\mu$g $^{32}$P-markierte Rekombinant-TRV-cDNS (Aktivität ca. 0,25 MBq).

Nach der Hybridisierung werden die Träger viermal bei Raumtemperatur jeweils 30 min in einer Lösung aus 3-fach-SSC und 0,1 % SDS gewaschen, luftgetrocknet und 2-5 Tage autoradiographiert. Die Auswertung der Röntgenfilme erfolgt visuell oder, nach Zerschneiden der Filme in Bahnen, mit einem Densitometer.

(b) Nachweis von BSMV-Virus (barley stripe mosaic virus) in Gerste mit cDNS

Die Aufbereitung der Proben erfolgt wie unter (a) beschrieben. Denaturiert wird die Virus-RNS durch Aufnehmen der in Alkohol gefällten Probe in einem Endvolumen von 10 $\mu$l, enthaltend 1 M Glyoxal und 10 mM $Na_{2/3}PO_4$ (pH 7,0), einstündiges Erhitzen auf 50 °C und anschließendes Abschrecken auf 0 °C. Hybridisierung, Waschen und Auswertung erfolgen wie unter (a) beschrieben. Die molekulare Sonde wird jedoch wie folgt gewonnen : An isolierter RNS von BSMV wird mit Hilfe von AMV-Reverse-Transcriptase und Oligo-dT als Primer sowie $^{32}$P-markierten $\alpha$-Desoxyribonucleotidtriphosphaten ein reverses Transkript produziert, das für die Hybridisierung eingesetzt werden kann.

Beispiel 17

(a) pH-Testpapier :

Ein erfindungsgemäßes Flachmaterial als Träger wird mit einer Lösung von 10 mg Methylrot und 30 mg Bromthylmolblau/100 ml Ethanol getränkt und getrocknet. Anschließend wird das Papier in Streifen von 0,5 cm Breite geschnitten und auf Kunststoffolie geklebt. Diese wird in Streifen von 0,5 cm Breite geschnitten, so daß sich auf jedem Teststreifen eine Reaktionszone von 0,5 × 0,5 cm befindet.

(b) Teststreifen zum Nachweis von Leukocyten im Urin :

Der flächige Träger wird mit 0,1 M Phosphatpuffer pH 8,0 und 2 mM EDTA imprägniert. Nach dem Trocknen erfolgt eine zweite Tränkung mit einer Lösung von 35 mg Indoxalacetat in 100 ml Aceton. Die Herstellung der Teststreifen erfolgt wie unter (a) beschrieben.

(c) Teststreifen zum Nachweis von Nitrit im Urin :

Der flächige Träger wird mit 0,5 M Citronensäurelösung mit einem pH-Wert von 1,6 imprägniert. Nach dem Trocknen erfolgt eine zweite Tränkung mit 5 mM Sulfanilsäureamid und 0,5 mM N-(1-Naphthyl)-ethylendiamindihydrochlorid in Aceton. Die Herstellung der Teststreifen erfolgt wie unter (a) beschrieben.

(d) Teststreifen zum Nachweis von reduzierenden Verbindungen im Urin :

Der flächige Träger wird mit einer Lösung von 125 mg 2,6-Dichlorphenolindophenol in 100 ml Sörensen-Phosphatpuffer mit einem pH-Wert von 7,0 imprägniert. Die Herstellung der Teststreifen erfolgt wie unter (a) beschrieben.

**Patentansprüche**

1. Makromolekulare Massen mit chemisch aktiven Füllstoffen, dadurch gekennzeichnet, daß sie bestehen aus

8

(I) 1 bis 80 Vol.-% einer makromolekularen Verbindung mit 4,6-Dihalogen-1,3,5-triazin-Gruppierungen,

(II) 0,5 bis 50 Vol.-% eines oder mehrerer 2,4,6-Trihalogen-1,3,5-triazine,

(III) bis zu 20 Vol.-% eines oder mehrerer Halogenide und

(IV) 5 bis 80 Vol.-% einer makromolekularen Verbindung, die mit der makromolekularen Verbindung (I) sowie den Füllstoffen verträglich ist, sowie gegebenenfalls

(V) bis zu 5 Vol.-% Puffersubstanzen und gegebenenfalls

(VI) einem flüssigen Dispergiermittel.

2. Makromolekulare Massen nach Anspruch 1, dadurch gekennzeichnet, daß die makromolekulare Verbindung (IV) ein Polymer mit nucleophilen Gruppen ist.

3. Makromolekulare Massen nach Anspruch 1, dadurch gekennzeichnet, daß die makromolekulare Verbindung (IV) ein Polymer ohne nucleophile Gruppen ist.

4. Makromolekulare Massen nach einem der Ansprüche 1 bis 3, dadurch gekennzeichnet, daß die makromolekulare Verbindung (I) mit 4,6-Dihalogen-1,3,5-triazin-Gruppierungen ein Polymer ist, das ggfs. teilweise noch nucleophile Gruppen aufweist.

5. Makromolekulare Massen nach einem der Ansprüche 1, 2 oder 4, dadurch gekennzeichnet, daß die makromolekulare Verbindung (I) und/oder die makromolekulare Verbindung (IV) natürliche Polymere wie Cellulose, Cellulosederivate, Dextrane oder Gelatine sind.

6. Makromolekulare Massen nach einem der Ansprüche 1, 2, 4 oder 5, dadurch gekennzeichnet, daß die makromolekulare Verbindung (I) und/oder die makromolekulare Verbindung (IV) synthetische Polymere sind, die OH-und/oder $NH_2$-Gruppen aufweisen.

7. Makromolekulare Massen nach Anspruch 6, dadurch gekennzeichnet, daß die makromolekulare Verbindung (I) und/oder die makromolekulare Verbindung (IV) Polyamide und/oder Polyvinylalkohole sind.

8. Makromolekulare Massen nach einem der Ansprüche 1 bis 7, dadurch gekennzeichnet, daß sie als disperse Systeme vorliegen.

9. Makromolekulare Massen nach Anspruch 8, dadurch gekennzeichnet, daß die Teilchen des dispersen Systems eine faserige, kugelförmige und/oder eckige Form besitzen.

10. Makromolekulare Massen nach einem der Ansprüche 1 bis 8, dadurch gekennzeichnet, daß sie als Formkörper vorliegen.

11. Makromolekulare Massen nach Anspruch 10, dadurch gekennzeichnet, daß die Formkörper eine kugelförmige, flächige oder eckige Form besitzen.

12. Makromolekulare Massen nach einem der Ansprüche 1 bis 9, dadurch gekennzeichnet, daß sie als Dispergiermittel (VI) ein inertes Lösungsmittel enthalten.

13. Makromolekulare Massen nach Anspruch 10 oder 11, dadurch gekennzeichnet, daß die Formkörper gesponnene Fäden sind.

14. Makromolekulare Massen nach einem der Ansprüche 1 bis 13, dadurch gekennzeichnet, daß die makromolekulare Verbindung ein Bindemittel ist.

15. Makromolekulare Massen nach einem der Ansprüche 10 bis 14, dadurch gekennzeichnet, daß die Formkörper eine papierähnliche Matrix besitzen.

16. Verfahren zur Herstellung der makromolekularen Massen mit chemisch aktiven Füllstoffen nach einem der Ansprüche 1 bis 15 durch Fixieren eines 2,4,6-Trihalogen-1,3,5-triazins auf den makromolekularen Anteilen der Masse, dadurch gekennzeichnet, daß das 2,4,6-Trihalogen-1,3,5-triazin durch mikrokristalline Abscheidung auf den makromolekularen Anteilen der Masse fixiert wird.

17. Verfahren nach Anspruch 16 durch Fixieren von 2,4,6-Trichlor-1,3,5-triazin auf Cellulosepartikeln, dadurch gekennzeichnet, daß die Cellulosepartikel zunächst mit 2,4,6-Trichlor-1,3,5-triazin behandelt werden, das 2,4,6-Trichlor-1,3,5-triazin unmittelbar darauf mit einer Mischung aus Essigsäure und einem mit Wasser mischbaren organischen Lösungsmittel bzw. Lösungsmittelgemisch mit 50 bis 90 Vol.-% organischem Lösungsmittel fixiert wird, und die Cellulosepartikel anschließend mit einem mit Wasser mischbaren organischen Lösungsmittel bzw. Lösungsmittelgemisch gewaschen werden.

18. Verfahren nach Anspruch 17, dadurch gekennzeichnet, daß als mit Wasser mischbare Lösungsmittel primäre Alkohole, wie Methanol, Ethanol, Propanol und Butanol, einschließlich ihrer Isomeren, und mit Wasser mischbare Ketone, wie Aceton, verwendet werden.

19. Verwendung der makromolekularen Massen nach einem der Ansprüche 1 bis 15 für Kapillar- und Elektroblotting, für Screening-Tests, zur Bindung von Mikroorganismen oder Zellen, in Festphasen-Immunoassays sowie als Träger für Teststreifen.

20. Verwendung der makromolekularen Massen gemäß Anspruch 19, wobei ein Trägermaterial verwendet wird, das aus Cellulose und/oder Cellulosederivaten mit 4,6-Dichlor-1,3,5-triazin-Gruppierungen in einer Menge von 1-80 Vol.-%, 2,4,6-Trichlor-1,3,5-triazin in einer Menge von 0,5-50 Vol.-%, Chloriden in einer Menge bis zu 20 Vol.-%, Cellulose und/oder Cellulosederivaten in einer Menge von 5-80 Vol.-% und gegebenenfalls aus Puffersubstanzen bis zu 5 Vol.-% besteht.

21. Verwendung der makromolekularen Massen nach einem der Ansprüche 1 bis 15 in Festphasen-Immunoassays zur immunologischen Bestimmung von Antigenen, Antikörpern, Haptenen, Viren, Hormonen und Pharmaka.

22. Verwendung der makromolekularen Massen gemäß Anspruch 21 unter Fixierung der komplementären Antikörper oder Antigene an cellulosehaltigen Materialien als feste Phase, Durchführung der Immunreaktion, Spaltung der gebildeten Antigen-Antikörper-Komplexe durch die Dissoziation herbeiführende Reagentien und Wiedereinsatz der beladenen festen Phase im Immunoassay, wobei als feste Phase zwei- oder dreidimensional nutzbare Formkörper verwendet werden, die aus Cellulose und/oder Cellulosederivaten mit 4,6-Dichlor-1,3,5-triazin-Gruppierungen in einer Menge von 1 bis 80 Vol.-% 2,4,6-Trihalogen-1,3,5-triazin in einer Menge von 0,5 bis 50 Vol.-%, Chloriden in einer Menge bis zu 20 Vol.-% und Cellulose und/oder Cellulosederivaten in einer Menge von 5 bis 80 Vol.-% bestehen.

23. Verwendung gemäß Anspruch 22, wobei die im Immunoassay eingesetzten dreidimensional nutzbaren Formkörper halbkugelförmige, kugelförmige, würfelförmige oder zylinderförmige Gestalt besitzen und ein Volumen von 0,01 bis 15 ml aufweisen.

24. Verwendung nach Anspruch 23, dadurch gekennzeichnet, daß kompakte oder hohle Formkörper verwendet werden.

25. Verwendung nach Anspruch 24, dadurch gekennzeichnet, daß offene hohle Formkörper verwendet werden.

26. Verwendung nach Anspruch 24, dadurch gekennzeichnet, daß geschlossene hohle Formkörper verwendet werden.

27. Verwendung gemäß einem der Ansprüche 21 bis 24, dadurch gekennzeichnet, daß als zweidimensional nutzbare Formkörper flächige Formkörper eingesetzt werden, deren Oberfläche in regelmäßigen und definierten Bereichen nutzbar ist, die eine Größe von 0,001 bis 5 cm$^2$ aufweisen.

28. Verwendung nach einem der Ansprüche 21 bis 27, dadurch gekennzeichnet, daß die Cellulose bzw. die Cellulose-derivate teilweise oder vollständig in Form faserförmiger Partikel eingesetzt werden.

29. Verwendung nach einem der Ansprüche 21 bis 28, dadurch gekennzeichnet, daß flächige Träger mit einer papierähnlichen Matrix eingesetzt werden.

30. Verwendung der makromolekularen Massen mit chemisch aktiven Füllstoffen nach einem der Ansprüche 1 bis 15 zum Nachweis von RNS pflanzliche Viren, Viroide, Virusoide bzw deren Abkömmlingen.

31. Verwendung gemäß Anspruch 30, wobei der Pflanzenextrakt auf einen flächigen Träger aufgetragen wird, der aus Cellulose und/oder Cellulosederivaten mit 4,6-Dichlor-1,3,5-triazin-Gruppierungen in einer Menge von 1-80 Vol.-%, 2,4,6-Trichlor-1,3,5-triazin in einer Menge von 0,5-50 Vol.-%, Halogeniden in einer Menge bis zu 20 Vol.-% und Cellulose und/oder Cellulosederivaten in einer Menge von 5-80 Vol.-% sowie ggfs. Puffersubstanzen in einer Menge bis zu 5 Vol.-% besteht, und der Nachweis der gebundenen RNS in an sich bekannter Weise durch Hybridisierung mit molekularen Sonden vorgenommen wird.

32. Verwendung der makromolekularen Massen nach einem der Ansprüche 1 bis 15 zur Herstellung von Teststreifen für den Nachweis von organischen und anorganischen Bestandteilen in Flüssigkeiten.

33. Verwendung gemäß Anspruch 32, wobei ein flächiger Träger verwendet wird, der aus Cellulose und/oder Cellulosederivaten mit 4,6-Dichlor-1,3,5-triazin-Gruppierungen in einer Menge von 1-80 Vol.-%, 2,4,6-Trichlor-1,3,5-triazin in einer Menge von 0,5-50 Vol.-%, Chloriden in einer Menge bis zu 20 Vol.-% und Cellulose und/oder Cellulosederivaten in einer Menge von 5-80 Vol.-% besteht.

34. Verwendung nach einem der Ansprüche 19 bis 33, dadurch gekennzeichnet, daß Cellulose bzw. Cellulosederivate eingesetzt werden, die teilweise oder vollständig aus faserförmigen Partikeln bestehen.

35. Verwendung nach einem der Ansprüche 19 bis 34, dadurch gekennzeichnet, daß flächige Träger mit einer papierähnlichen Matrix eingesetzt werden.

**Claims**

1. Macromolecular masses with chemically-active filling materials, characterised in that they consist of

(I) 1 to 80 vol.% of a macromolecular compound with 4,6-dihalo-1,3,5-triazine groupings,

(II) 0.5 to 50 vol.% of one or more 2,4,6-trihalo-1,3,5-triazines,

(III) up to 20 vol.% of one or more halides and

(IV) 5 to 80 vol.% of a macromolecular compound which is compatible with the macromolecular compound (I), as well as the filling materials, as well as possibly

(V) up to 5 vol.% of buffer substances and possibly

(VI) a liquid dispersion agent.

2. Macromolecular masses according to claim 1, characterised in that the macromolecular compound (IV) is a polymer with nucleophilic groups.

3. Macromolecular masses according to claim 1, characterised in that the macromolecular compound (IV) is a polymer without nucleophilic groups.

4. Macromolecular masses according to one of claims 1 to 3, characterised in that the macromolecular compound (I) with 4,6-dihalo-1,3,5-triazine groupings is a polymer which possibly also has nucleophilic groups.

5. Macromolecular masses according to one of claims 1, 2 or 4, characterised in that macromolecular compound (I) and/or the macromolecular compound (IV) are natural polymers, such as cellulose, cellulose derivatives, dextrans or gelatine.

6. Macromolecular masses according to one of claims 1, 2, 4 or 5, characterised in that the macromolecular compound (I) and/or macromolecular compound (IV) are synthetic polymers which have OH and/or NH$_2$ groups.

7. Macromolecular masses according to claim 6, characterised in that the macromolecular compound (I) and/or the macromolecular compound (IV) are polyamides and/or polyvinyl alcohols.

8. Macromolecular masses according to one of claims 1 to 7, characterised in that they are present as dispersed systems.

9. Macromolecular masses according to claim 8, characterised in that the particles of the dispersed system possess a fibrous, spheroidal and/or angular form.

10. Macromolecular masses according to one of claims 1 to 8, characterised in that they are present as formed bodies.

11. Macromolecular masses according to claim 10, characterised in that the formed bodies possess a spheroidal, planar or angular shape.

12. Macromolecular masses according to one of claims 1 to 9, characterised in that they contain an inert solvent as dispersion agent (VI).

13. Macromolecular masses according to claim 10 or 11, characterised in that the formed bodies are spun filaments.

14. Macromolecular masses according to one of claims 1 to 13, characterised in that the macromolecular compound is a binding agent.

15. Macromolecular masses according to one of claims 10 to 14, characterised in that the formed bodies possess a paper-like matrix.

16. Process for the production of macromolecular bodies with chemically active filling materials according to one of claims 1 to 15 by fixing of a 2,4,6-trihalo-1,3,5-triazine to the macromolecular components of the mass, characterised in that the 2,4,6-trihalo-1,3,5-triazine is fixed by microcrystalline deposition on the macromolecular components of the mass.

17. Process according to claim 16 by fixing of 2,4,6-trichloro-1,3,5-triazine on to cellulose particles, characterised in that the cellulose particles are first treated with 2,4,6-trichloro-1,3,5-triazine, the 2,4,6-trichloro-1,3,5-triazine is immediately thereafter fixed with a mixture of acetic acid and an organic solvent miscible with water or organic solvent mixture with 50 to 90 vol.% organic solvent and the cellulose particles subsequently washed with a water-miscible organic solvent or solvent mixture.

18. Process according to claim 17, characterised in that, as solvents miscible with water, there are used primary alcohols, such as methanol, ethanol, propanol and butanol, including their isomers, and ketones miscible with water, such as acetone.

19. Use of macromolecular masses according to one of claims 1 to 15 for capillary and electro-blotting, for screening tests, for the binding of micro-organisms or cells, in solid phase immunoassays, as well as carriers for test strips.

20. Use of the macromolecular masses according to claim 19, whereby a carrier material is used which consists of cellulose and/or cellulose derivatives with 4,6-Dichloro-1,3,5-triazine groupings in an amount of 1-80 vol.%, 2,4,6-trichloro-1,3,5-triazine in an amount of 0.5-50 vol.%, chlorides in an amount of up to 20 vol.%, cellulose and/or cellulose derivatives in an amount of 5-80 vol.% and possibly of buffer substances up to 5 vol.%.

21. Use of the macromolecular masses according to one of claims 1 to 15 in solid phase immunoassays for the immunological determination of antigens, antibodies, haptens, viruses, hormones and pharmaceuticals.

22. Use of the macromolecular masses according to claim 21 for the fixing of the complementary antibodies or antigens to cellulose-containing materials as solid phase, carrying out of the immune reaction, cleavage of the antigen-antibody complexes formed by reagents bringing about the dissociation and using again of the loaded solid phase in the immuno assay, whereby, as solid phase, two- or three-dimensionally useable formed bodies are used which consist of cellulose and/or cellulose derivatives with 4,6-dichloro-1,3,5-triazine groupings in an amount of 1 to 80 vol.%, 2,4,6-trihalo-1,3,5-triazine in an amount of 0.5 to 50 vol.%, chlorides in an amount of up to 20 vol'% and cellulose and/or cellulose derivatives in an amount of 5 to 80 vol.%.

23. Use according to claim 22, whereby the three-dimensionally useable formed bodies used in the immunoassay possess a hemispheroidal, spheroidal, cubic or cylinder-shaped form and have a volume of 0.01 to 15 ml.

24. Use according to claim 23, characterised in that compact or hollow formed bodies are used.

25. Use according to claim 24, characterised in that open hollow formed bodies are used.

26. Use according to claim 24, characterised in that closed hollow formed bodies are used.

27. Use according to one of claims 21 to 24, characterised in that, as two-dimensionally useable formed bodies, planar formed bodies are used, the surface of which is useable in regular and defined regions which have a size of 0.001 to 5 cm$^2$.

28. Use according to one of claims 21 to 27, characterised in that the cellulose or cellulose derivatives are used partly or completely in the form of fibre-shaped particles.

29. Use according to one of claims 21 to 28, characterised in that planar carriers with a paper-like matrix are used.

30. Use of the macromolecular masses with chemically active filling materials according to one of claims 1 to 15 for the detection of RNA of plant viruses, viroids, virusoids or their derivatives.

31. Use according to claim 30, whereby the plant extract is applied to a planar carrier which consists of cellulose and/or cellulose derivatives with 4,6-dichloro-1,3,5-triazine groupings in an amount of 1-80 vol.%, 2,4,6-trichloro-1,3,5-triazine in an amount of 0.5-50 vol.%, halides in an amount of up to 20 vol.% and cellulose and/or cellulose derivatives in an amount of 5-80 vol.%, as well as possibly buffer substances in an amount of up to 5 vol.% and the detection of the bound RNA is carried out in per se known manner by hybridisation with molecular probes.

32. Use of the macromolecular masses according to one of claims 1 to 15 for the production of test strips for the detection of organic and inorganic components of liquids.

33. Use according to claim 32, whereby a planar carrier is used which consists of cellulose and/or cellulose derivatives with 4,6-dichloro-1,3,5-triazine groupings in an amount of 1-80 vol.%, 2,4,6-trichloro-1,3,5-triazine in an amount of 0.5-50 vol.%, chlorides in an amount of up to 20 vol.% and cellulose and/or cellulose derivatives in an amount of 5-80 vol.%.

34. Use according to one of claims 19 to 33, characterised in that cellulose or cellulose derivatives are used which consist partly or completely of fibre-shaped particles.

35. Use according to one of claims 19 to 34, characterised in that planar carriers are used with a paper-like matrix.

**Revendications**

1. Masses macromoléculaires avec matières de charge chimiquement actives, caractérisées en ce qu'elles se composent de
(I) 1 à 80 % en volume d'un composé macromoléculaire avec groupements 4,6-dihalogéno-1,3,5-triazine,
(II) 0,5 à 50 % en volume d'une ou plusieurs 2,4,6-trihalogéno-1,3,5-triazines,
(III) jusqu'à 20 % en volume d'un ou plusieurs halogénures et
(IV) 5 à 80 % en volume d'un composé macromoléculaire qui est compatible avec le composé macromoléculaire (I) et aussi avec les matières de charge,
de même qu'éventuellement
(V) jusqu'à 5 % en volume de substances tampons
et éventuellement
(VI) un agent dispersant liquide.

2. Masses macromoléculaires selon la revendication 1, caractérisées en ce que le composé macromoléculaire (IV) est un polymère avec groupes nucléophiles.

3. Masses macromoléculaires selon la revendication 1, caractérisées en ce que le composé macromoléculaire (IV) est un polymère sans groupes nucléophiles.

4. Masses macromoléculaires selon l'une des revendications 1 à 3, caractérisées en ce que le composé macromoléculaire (I) avec groupements 4,6-dihalogéno-1,3,5-triazine est un polymère qui en partie présente éventuellement encore des groupes nucléophiles.

5. Masses macromoléculaires selon l'une des revendications 1, 2 ou 4, caractérisées en ce que le composé macromoléculaire (I) et/ou le composé macromoléculaire (IV) sont des polymères naturels comme la cellulose, les dérivés de cellulose, les dextranes ou les gélatines.

6. Masses macromoléculaires selon l'une des revendications 1, 2, 4 ou 5, caractérisées en ce que le composé macromoléculaire (I) et/ou le composé macromoléculaire (IV) sont des polymères synthétiques qui présentent des groupes OH et/ou NH$_2$.

7. Masses macromoléculaires selon la revendication 6, caractérisées en ce que le composé macromoléculaire (I) et/ou le composé macromoléculaire (IV) sont des polyamides et/ou des alcools polyvinyliques.

8. Masses macromoléculaires selon l'une des revendications 1 à 7, caractérisées en ce qu'elles se présentent sous la forme de systèmes dispersés.

9. Masses macromoléculaires selon la revendication 8, caractérisées en ce que les particules du système dispersé possèdent une forme fibreuse, sphérique et/ou anguleuse.

10. Masses macromoléculaires selon l'une des revendications 1 à 8, caractérisées en ce qu'elles se présentent sous forme de corps moulés.

11. Masses macromoléculaires selon la revendication 10, caractérisées en ce que les corps moulés possèdent une forme sphérique, plane ou anguleuse.

12. Masses macromoléculaires selon l'une des revendications 1 à 9, caractérisées en ce qu'elles contiennent comme agent dispersant (VI) un solvant inerte.

13. Masses macromoléculaires selon la revendication 10 ou 11, caractérisées en ce que les corps moulés sont des filaments filés.

14. Masses macromoléculaires selon l'une des revendications 1 à 13, caractérisées en ce que le composé macromoléculaire est un agent liant.

15. Masses macromoléculaires selon l'une des revendications 10 à 14, caractérisées en ce que les corps moulés possèdent une matrice semblable à du papier.

16. Procédé de fabrication des masses macromoléculaires avec matières de charge chimiquement actives selon l'une des revendications 1 à 15 par fixation d'une 2,4,6-trihalogéno-1,3,5-triazine sur les portions macromoléculaires de la masse, caractérisé en ce qu'on fixe la 2,4,6-trihalogéno-1,3,5-triazine par dépôt microcristallin sur les portions macromoléculaires de la masse.

17. Procédé selon la revendication 16 par fixation de la 2,4,6-trichloro-1,3,5-triazine sur des particules de cellulose, caractérisé en ce qu'on traite les particules de cellulose tout d'abord avec de la 2,4,6-trichloro-1,3,5-triazine, on fixe directement après cela la 2,4,6-trichloro-1,3,5-triazine avec un mélange d'acide acétique et un solvant organique ou mélange de solvants organiques miscibles avec l'eau à 50 à 90 % en volume de solvant organique, et on lave ensuite les particules de cellulose avec un solvant organique ou mélange de solvants organiques miscibles avec l'eau.

18. Procédé selon la revendication 17, caractérisé en ce qu'on utilise comme solvants miscibles avec l'eau des alcools primaires comme le méthanol, l'éthanol, le propanol et le butanol, y compris leurs isomères et des cétones miscibles avec l'eau, comme l'acétone.

19. Utilisation des masses macromoléculaires selon l'une des revendications 1 à 15 pour l'absorption capillaire et l'électro-absorption, pour des tests de triage (screening), pour retenir les microorganismes ou les cellules, dans les immuno-essais en phase solide de même que comme supports pour rubans d'essai.

20. Utilisation des masses macromoléculaires selon la revendication 19, dans laquelle on emploie un matériel de support qui se compose de cellulose et/ou de dérivés de cellulose avec groupements 4,6-dichloro-1,3,5-triazine en une quantité de 1 à 80 % en volume, de 2,4,6-trichloro-1,3,5-triazine en une quantité de 0,5 à 50 % en volume, de chlorures en une quantité allant jusqu'à 20 % en volume, de cellulose et/ou de dérivés de cellulose en une quantité de 5 à 80 % en volume et éventuellement de substances tampons jusqu'à concurrence de 5 % en volume.

21. Utilisation des masses macromoléculaires selon l'une des revendications 1 à 15 dans des immuno-essais en phase solide pour la détermination immunologique d'antigènes, d'anticorps, d'haptènes, de virus, d'hormones et de pharmacas.

22. Utilisation des masses macromoléculaires selon la revendication 21 avec fixation des anticorps ou antigènes complémentaires sur des matériels renfermant de la cellulose en tant que phase solide, exécution de l'immunoréaction, clivage des complexes antigène-anticorps formés par des réactifs produisant la dissociation et réutilisation de la phase solide chargée dans l'immuno-essai, en utilisant comme phase solide des corps moulés bi- ou tridimensionnellement utilisables qui consistent en de la cellulose et/ou des dérivés de cellulose avec groupements 4,6-dichloro-1,3,5-triazine en une quantité de 1 à 80 % en volume, en de la 2,4,6-trihalogéno-1,3,5-triazine en une quantité de 0,5 à 50 % en volume, en des chlorures en une quantité allant jusqu'à 20 % en volume et en de la cellulose et/ou des dérivés de cellulose en une quantité de 5 à 80 % en volume.

23. Utilisation selon la revendication 22, dans laquelle les corps moulés tridimensionnellement utilisables dans l'immuno-essai possèdent une forme hémisphérique, sphérique, cubique ou cylindrique et présentent un volume de 0,01 à 15 ml.

24. Utilisation selon la revendication 23, caractérisée en ce qu'on utilise des corps moulés compacts ou creux.

25. Utilisation selon la revendication 24, caractérisée en ce qu'on utilise des corps moulés creux ouverts.

26. Utilisation selon la revendication 24, caractérisée en ce qu'on utilise des corps moulés creux fermés.

27. Utilisation selon l'une des revendications 21 à 24, caractérisée en ce qu'on utilise comme corps moulés bidimensionnellement utilisables des corps moulés plats dont la surface est utilisable dans des zones régulières et définies qui présentent une dimension de 0,001 à 5 cm$^2$.

28. Utilisation selon l'une des revendications 21 à 27, caractérisée en ce qu'on utilise la cellulose ou des dérivés de cellulose partiellement ou totalement sous la forme de particules fibreuses.

29. Utilisation selon l'une des revendications 21 à 28, caractérisée en ce qu'on utilise des supports plats ayant une matrice semblable à du papier.

30. Utilisation des masses macromoléculaires avec des matières de charge chimiquement actives suivant les revendications 1 à 15 pour la détermination de l'ARN de virus, viroïdes, virusoïdes végétaux ou de leurs dérivés.

31. Utilisation selon la revendication 30, dans laquelle l'extrait végétal est appliqué sur un support plat qui consiste en de la cellulose et/ou en des dérivés de cellulose avec groupements 4,6-dichloro-1,3,5-triazine en une quantité de 1 à 80 % en volume, en de la 2,4,6-trichloro-1,3,5-triazine en une quantité de 0,5 à 50 % en volume, en des halogénures en une quantité allant jusqu'à 20 % en volume et en de la cellulose et/ou des dérivés de cellulose en une quantité de 5 à 80 % en volume, de même qu'éventuellement en des substances tampons en une quantité allant jusqu'à 5 % en volume, et dans laquelle la détermination de l'ARN fixé est entreprise d'une manière connue en elle-même par hybridation avec des sondes moléculaires.

32. Utilisation des masses macromoléculaires suivant l'une des revendications 1 à 15 pour la fabrication de rubans d'essai pour la détermination de constituants organiques et minéraux dans des liquides.

33. Utilisation selon la revendication 32, dans laquelle on utilise un support plat qui consiste en de la cellulose et/ou en des dérivés de cellulose avec groupements 4,6-dichloro-1,3,5-triazine en une quantité de 1 à 80 % en volume, en de la 2,4,6-trichloro-1,3,5-triazine en une quantité de 0,5 à 50 % en volume, en des chlorures en une quantité allant jusqu'à 20 % en volume, et en de la cellulose et/ou des dérivés de cellulose en une quantité de 5 à 80 % en volume.

34. Utilisation selon l'une des revendications 19 à 33, caractérisée en ce qu'on utilise de la cellulose ou des dérivés de cellulose qui consistent partiellement ou totalement en des particules fibreuses.

35. Utilisation selon l'une des revendications 19 à 34, caractérisée en ce qu'on utilise des supports plats ayant une matrice semblable à du papier.